# EUROPEAN PATENT SPECIFICATION

(11) **EP 3 122 724 B1**
(45) Date of publication and mention of the grant of the patent: **28.02.2018**
(21) Application number: 15715961.7
(22) Date of filing: 24.03.2015
(51) Int. Cl.: C07D 209/52

(54) **A PROCESS FOR PRODUCING SAXAGLIPTIN**
VERFAHREN ZUR HERSTELLUNG VON SAXAGLIPTIN
PROCÉDÉ DE PRODUCTION DE SAXAGLIPTINE

(30) Priority: 24.03.2014 CZ 20140177
(43) Date of publication of application: 01.02.2017
(73) Proprietor: Zentiva K.S., 102 37 Praha 10 (CZ)
(72) Inventor: CERNY, Josef, 27401 Slany (CZ); HALAMA, Ales, 530 09 Pardubice (CZ); STACH, Jan, 190 16 Praha 9 (CZ)
(74) Representative: Jirotkova, Ivana
(86) International application number: PCT/CZ2015/000023
(87) International publication number: WO 2015/144098

(56) References cited:
- WO-A2-01/68603
- WO-A2-2006/020664
- US-A1- 2005 267 191
- US-A1- 2011 275 687

## Description

### Technical Field

The invention relates to an improved process for producing the anti-diabetic drug saxagliptin of formula **I,** 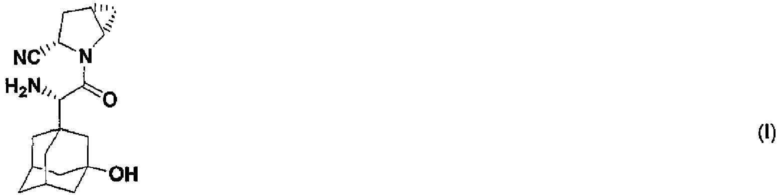 which is a new and efficient dipeptidyl peptidase - 4 inhibitor (DPP-4), suitable for combination treatment where monotherapy with oral anti-diabetic drugs fails. The active substance is contained in the marketed drug form Onglyza by Bristol-Myers Squibb.

### Background Art

Synthesis of saxagliptin (Scheme I) was described in the basic patent of 2001 (WO 0168603). The process comprises protection of the intermediate of formula II with the triethylsilyl group, dehydration of the resulting intermediate product with phosphorus oxychloride in the presence of imidazole in pyridine and deprotection of the Boc (tert-butyloxycarbonyl) protecting group, producing saxagliptin. The use of the expensive triethylsilyl triflate at a very low temperature (-78°C) and of the chromatographic isolation of the resulting product is a disadvantage. 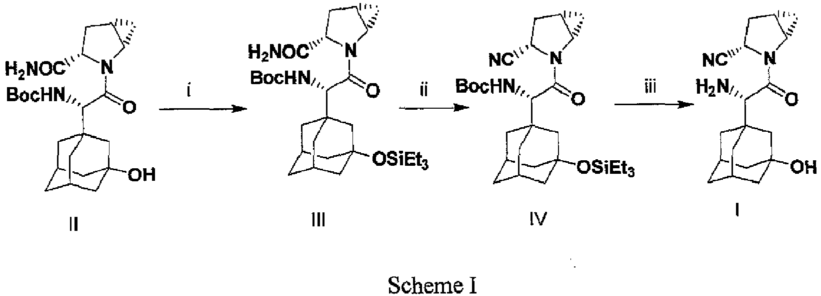 The new process of Bristol-Myers Squibb uses trifluoroacetic anhydride as a protecting agent and at the same time as an agent for dehydration of the amide to the nitrile (WO 2006020664, Scheme II)

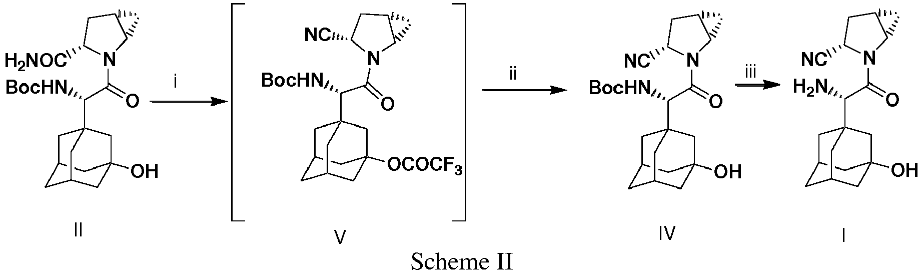

The improved process does not require a low temperature, but it uses trifluoroacetic anhydride producing trifluoroacetic acid after the hydrolysis, which is a major pollutant and whose use in the industrial production should be reduced to the necessary minimum (Boutonnet JC, Bingham P, Calamari D, de Rooij C, Franklin J, Kawano T, Libre J-M, McCulloch A, Malinvemo G, Odom JM, Rusch GM, Smythe K, Sobolev I, Thompson R, Tiedje JM (1999). "Environmental risk assessment of trifluoroacetic acid". International Journal of Human and Ecological Risk Assessment 5 (1): 59-124.).

### Disclosure of Invention

This invention provides an improved process for producing saxagliptin of formula **I,** 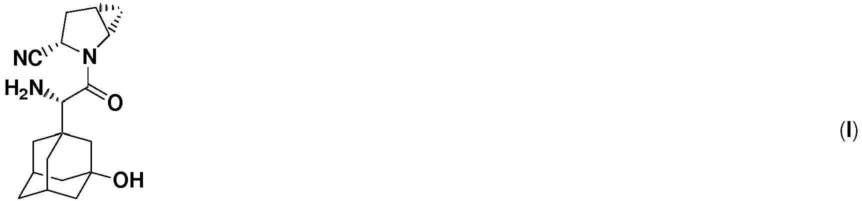 starting from the intermediate of formula **II,** 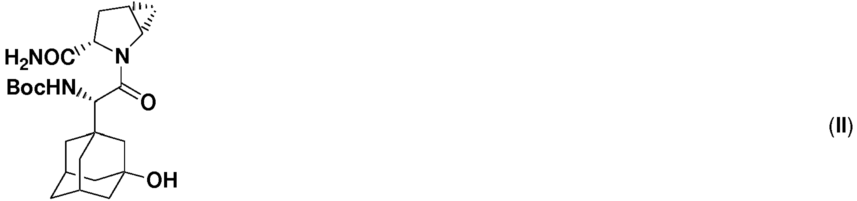 wherein Boc is tert-butyloxycarbonyl, with a silylating agent in the presence of a base, followed by dehydration of the resulting intermediate with the use of phosphorus oxychloride in the presence of imidazole and a base, producing Boc-saxagliptin of formula IV 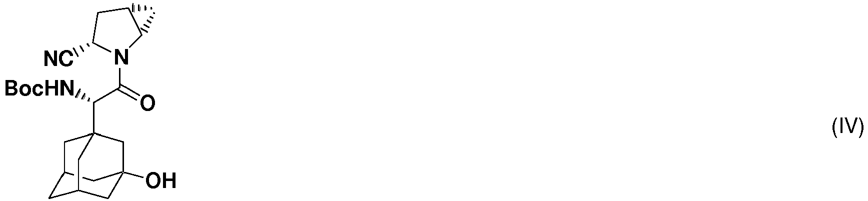 which is converted to saxagliptin of formula I by deprotection in an acidic environmnent.

The improvement of the present invention resides in the fact that a silylating agent which introduces the trimethylsilyl group is used, namely a silylating agent selected from the group comprising bis(trimethylsilyl)acetamide, trimethylsilyl chloride, trimethylsilyl triflate, and bis(trimethylsilyl)trifluoroacetamide.

Detailed investigation of the above mentioned basic patent (WO 0168603) has shown that reproduction of Example 60, step 3 on page 89, only provides a mixture of substances wherein only about 40% by weight of the starting substance is silylated and the rest consists of the starting substance and other impurities. After chromatography just a low yield (30% by weight) of the product can be obtained, which is further dehydrated with a mixture of phosphorus oxychloride in the presence of imidazole and pyridine, which provides an impure silylated product.

In our case bis(trimethylsilyl)acetamide is used for the protection, providing the protected intermediate, which, however, cannot be isolated due to its instability, this being, on the other hand, a considerable advantage for the subsequent reaction, where, after dehydration, the protecting group is released by the mere processing of the reaction. Then, the resulting crude reaction mixture is purer than in the case of using any protecting agent, which remains in the molecule after the dehydration and must be removed, e.g., in an acidic or alkaline environment at an elevated temperature. If any other silylating agents are used, the reaction mixture is surprisingly less pure. The table below shows purity of the reaction mixture depending on the silylating agents used (Table1).

**Table 1:**

| Agent | HPLC of the reaction mixture (%) | Base | Temperature (°C) |
|---|---|---|---|
| bis(trimethylsilyl)acetamide | 95.6 | pyridine | 25 |
| trimethylsilyl chloride | 91.4 | pyridine | 25 |
| trimethylsilyl triflate | 87.4 | pyridine | 25 |
| bis(trimethylsilyl)trifluoroacetamide | 85.6 | pyridine | 25 |

The effect of the base on the purity of the reaction mixture is also surprising as pyridine clearly provides the best results of the bases used (Table 2).

**Table 2:**

| Agent | HPLC of the reaction mixture (%) | Base | Temperature (°C) |
|---|---|---|---|
| bis(trimethylsilyl)acetamide | 95.6 | pyridine | 25 |
| bis(trimethylsilyl)acetamide | 88.4 | ethyl diisopropylamine (EDIPA) | 25 |
| bis(trimethylsilyl)acetamide | 84.4 | triethylamine | 25 |
| bis(trimethylsilyl)acetamide | 60.7 | ethyl nicotinate | 25 |

The surprising effect can be explained by the synergistic action of the base and silylating agent used.

The reactions were routinely monitored by means of HPLC using a HP 1050 device equipped with a Phenomenex Luna 5µ C18(2) column, 250 x 4.6 mm, fitted with a 210 nm UV detector. Phase A: 1.74 g of K₂HPO₄/11 of water (pH = 7.5; Phase B: acetonitrile).

The invention is further elucidated in the working examples below. The examples, which illustrate the improvement of the process of the invention, only have an illustrative character and do not restrict the scope of the invention in any respect.

### Examples

### Example 1:

(S)-N-Boc-3-hydroxyadamantylglycuie-L-cis-4,5-methanoprolinamide of formula **II** (15 g; 34.6 mmol) is dissolved in 75 ml of acetonitrile. Pyridine (6.2 ml; 76.1 mmol) is added to the resulting solution. Then, BSA (16.9 ml; 69.2 mmol) is added dropwise under cooling at such a rate that the temperature in the reaction mixture does not exceed 30°C. The reaction mixture is agitated for 1 hour. Then, imidazole (7.1 g; 103.8 mmol) and pyridine (16.8 ml; 207.6 mmol) are added. After 5 minutes of agitation POCl₃ (9.7 ml; 103.8 mmol) is added dropwise and the reaction mixture is agitated at the room temperature for 3 hours. Water (100 ml) is added dropwise to the resulting suspension and the product is extracted with ethyl acetate (3 x 150 ml). The combined organic extracts are gradually washed with 1 M HCl (40 ml), a 24% solution of K₂CO₃ (40 ml) and a saturated solution of NaCl (40 ml). The organic solvents are evaporated in a vacuum rotary evaporator. The crude product is crystallized from a mixture of acetonitrile (45 ml) and water (210 ml). The separated substance is aspirated and washed with water (30 ml). The final product is then dried in a vacuum drier at 40°C. 10.8 g (75% by weight of the theoretical quantity) of Boc-saxagliptin (**IV**) was obtained.

### Example 2:

*N-*Boc-3-hydroxy-1-adamantyl-*D*-glycine (60 g; 184.4 mmol), *cis*-4,5-methane prolinamide methane sulfonate (43 g; 193.6 mmol), hydroxybenzotriazole hydrate (28 g; 180.7 mmol) and 1-ethyl-3-(3-dimethylaminopropyl)carbodiimide hydrochloride (39 g; 201.0 mmol) are charged into a reactor. A previously prepared solution of ethyl diisopropylamine (71 ml, 405.7 mmol), acetonitrile (180 ml) and ethyl acetate (60 ml) is added to the solid substances. The reaction mixture is heated up to 40°C and agitated for 3 hours. Ethyl acetate (540 ml), 2 M HCl (92 ml), water (92 ml) and brine (160 ml) are gradually added into the reaction mixture. The whole mixture is agitated for 20 minutes. Then, the aqueous layer is separated. The organic layer is washed with a 20% solution of KHCO₃ (2 x 300 ml). The solution is concentrated by means of vacuum distillation to the volume of 180 ml. After addition of acetonitrile (120 ml) the distillation is continued while acetonitrile (450 ml) is continuously added at such a rate to maintain a constant volume in the reactor. After completion of the distillation pyridine (33 ml; 405.7 mmol) is added to the solution. Then, BSA (90 ml; 368.8 mmol) is added dropwise under cooling at such a rate that the temperature in the reaction mixture does not exceed 30°C. The reaction mixture is agitated for 1 hour, producing the silylated intermediate of formula **II**. POCl₃ (52 ml; 553.2 mmol) and acetonitrile (50 ml) are charged into another reactor. A solution of imidazole (38 g; 553.2 mmol) and pyridine (89 ml; 1106.3 mmol) in acetonitrile (30 ml) is added to the solution of POCl₃ dropwise under cooling. After 5 minutes of agitation the solution of the silylated intermediate of formula **II** is added dropwise and the reaction mixture is agitated at the room temperature for 3 hours. Then, the solid substance is filtered off and the solution of the product is added to water (400 ml) dropwise. The product is extracted with ethyl acetate (3 x 250 ml). The combined organic extracts are gradually washed with 2 M HCl (100 ml), a 8% solution of NaHCO₃ (100 ml). The organic solvents are evaporated in a vacuum rotary evaporator. The crude product is crystallized from a mixture of isopropanol (180 ml) and water (600 ml). The separated substance is aspirated and washed with a solution of isopropanol/water (3/1; 100 ml). The final product is then dried in a vacuum drier at 40°C. 59.8 g (78% by weight of the theoretical quantity) of Boc-saxagliptin of formula **IV** was obtained.

### Example 3:

Boc-Saxagliptin of formula **IV** (40 g; 96.3 mmol) is suspended in 40 ml of isopropanol and 40 ml of water. Trifluoroacetic acid (1.5 ml; 19.3 mmol) is added and the whole mixture is heated up to 65°C. Trifluoroacetic acid (28 ml; 365.8 mmol) is added dropwise and the whole mixture is agitated at 65°C for 4 hours. The reaction mixture is cooled down to 25°C and water (80 ml) and dichloromethane (240 ml) are added. A 10M solution of NaOH (18.3 ml) and a 24% solution of K₂CO₃ (18 ml) are gradually added to the vigorously agitated emulsion dropwise. After addition of NaCl (63 g) the whole mixture is stirred for 30 minutes. The layers are separated and the organic layer is concentrated to the volume of 125 ml. After addition of ethyl acetate (40 ml) the solution is filtered. 40 ml of ethyl acetate and 1.1 ml of water are added to the clear filtrate and the reaction mixture is agitated for 30 minutes. 1.1 ml of water is added to the suspension, which is agitated for another 20 minutes. This is followed by addition of 8.5 ml of water. All dichloromethane is replaced with ethyl acetate by means of vacuum distillation, ethyl acetate being continuously added. After completion of the distillation water (3 ml) is added and the suspension is cooled down to 5°C. The separated substance is aspirated and washed with wet ethyl acetate (2 ml of water and 80 ml of ethyl acetate). The final product is then dried in a vacuum drier at 40°C. 26.6 g (83% by weight of the theoretical quantity) of saxagliptin monohydrate of formula **I** was obtained.

### Example 4:

Boc-Saxagliptin (IV) (5 g; 12.0 mmol) is suspended in 5 ml of isopropanol and 5 ml of water. Methanesulfonic acid (1.09 ml; 16.8 mmol) is added and the whole mixture is heated up to 65°C. The whole mixture is agitated at 65°C for 3 hours. The reaction mixture is cooled down to 25°C and water (10 ml) and dichloromethane (30 ml) are added. A 10 M solution of NaOH (0.760 ml) and a 24% solution of K₂CO₃ (1.13 ml) are gradually added to the vigorously agitated emulsion dropwise. After addition of NaCl (6.2 g) the whole mixture is agitated for 30 minutes. The layers are separated and the organic layer is evaporated. The product is crystallized from a mixture of ethyl acetate (15 ml) and water (0.75 ml). 3.1 g (78% by weight of the theoretical quantity) of saxagliptin monohydrate (I) was obtained.

## Claims

1. A process for producing saxagliptin of formula **I** 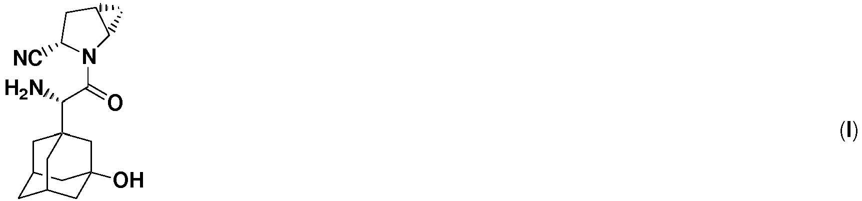 that comprises a reaction of the intermediate of formula **II,** 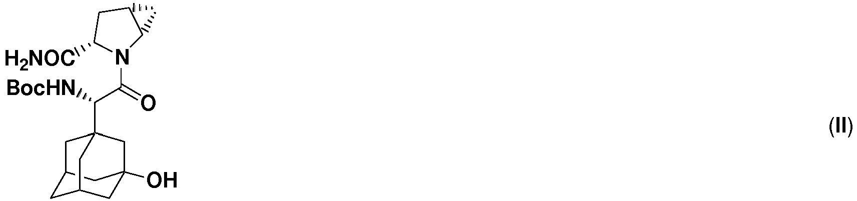 wherein Boc is tert-butyloxycarbonyl,
with a silylating agent in the presence of a base, further dehydration of the resulting intermediate with the use of phosphorus oxychloride in the presence of imidazole and a base, producing Boc-saxagliptin of formula IV, 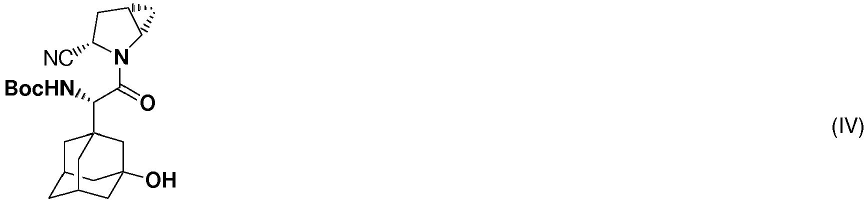 which is converted to saxagliptin of formula **I** by deprotection in an acidic environment, **characterized in that** a silylating agent, selected from the group comprising bis(trimethylsilyl)acetamide, trimethylsilyl chloride, trimethylsilyl triflate, and bis(trimethylsilyl)trifluoroacetamide, is used.

2. The process according to claim 1, **characterized in that** the silylating agent is bis(trimethylsilyl)acetamide.

3. The process according to claims 1 or 2, **characterized in that** the base is selected from the group comprising pyridine, ethyl diisopropylamine, triethylamine and ethyl nicotinate.

4. The process according to claim 3, **characterized in that** the base is pyridine.

## Patentansprüche

1. Verfahren zur Herstellung von Saxagliptin der Formel I, 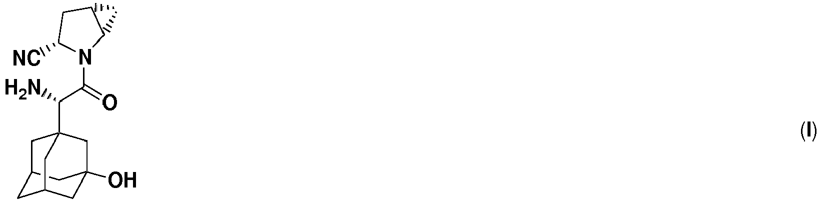 welches eine Reaktion der Zwischenstufe der Formel II umfasst, 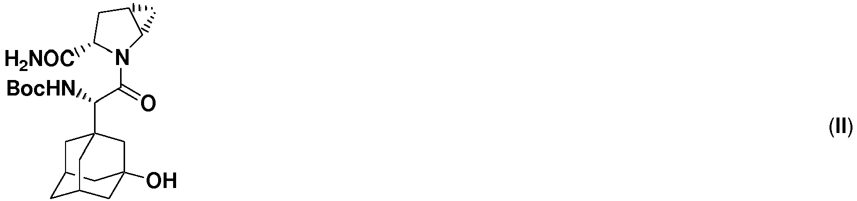 worin Boc tert-Butyloxycarbonyl ist,
mit einem Silylierungsmittel in Gegenwart einer Base, weiter Dehydration der entstehenden Zwischenstufe unter Verwendung von Phosphoroxychlorid in Gegenwart von Imidazol und einer Base, wobei Boc-Saxagliptin der Formel IV entsteht, 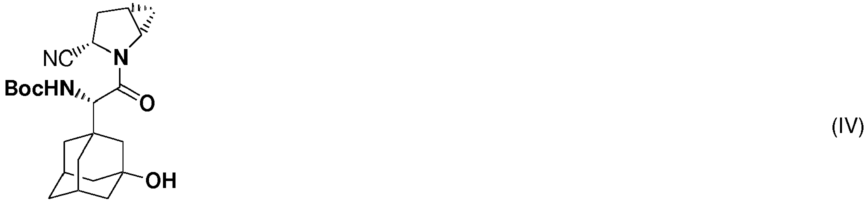 welches in einer sauren Umgebung durch Entschützung zu Saxagliptin der Formel I umgewandelt wird, dadurch gekenn-zeichnet, dass ein Silylierungsmittel verwendet wird, welches gewählt ist aus der Gruppe, die Bis(trimethylsilyl)acetamid, Trimethylsilylchlorid, Trimethylsilyltriflat und Bis(trimethylsilyl)trifluoroacetamid umfasst.

2. Verfahren nach Anspruch 1, **dadurch gekennzeichnet, dass** das Silylierungsmittel Bis(trimethylsilyl)acetamid ist.

3. Verfahren nach Anspruch 1 oder 2, **dadurch gekennzeichnet, dass** die Base ausgewählt ist aus der Gruppe, die Pyridin, Ethyldiisopropylamin, Triethylamin und Ethylnicotinat umfasst.

4. Verfahren nach Anspruch 3, **dadurch gekennzeichnet, dass** die Base Pyridin ist.

## Revendications

1. Un procédé de production de saxagliptine de formule I 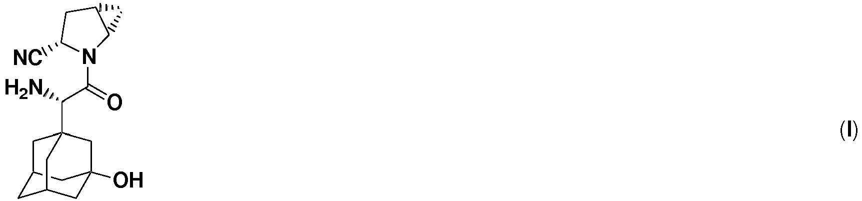 qui comprend:
- une réaction de l'intermédiaire de formule II, avec un agent de silylation en présence d'une base, 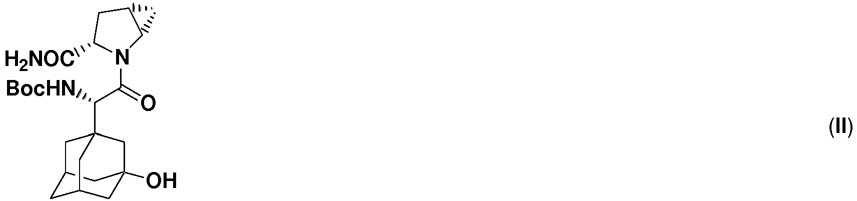 où Boc est tert-butyloxycarbonyle,
- puis une déshydratation de l'intermédiaire résultant avec l'utilisation d'oxychlorure de phosphore en présence d'imidazole et d'une base, produisant du Boc-saxagliptine de formule IV, 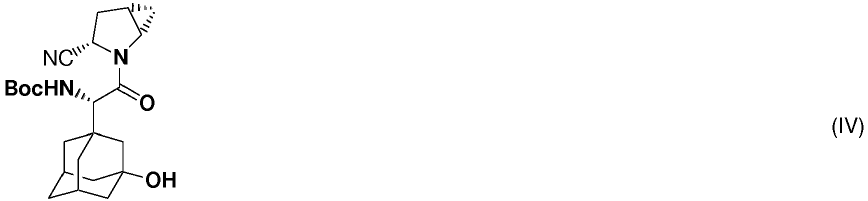
qui est converti en saxagliptine de formule I par déprotection en milieu acide, **caractérisé en ce que** l'on utilise un agent de silylation, choisi dans le groupe comprenant le bis (triméthylsilyl) acétamide, le chlorure de triméthylsilyle, le triflate de triméthylsilyle et le bis (triméthylsilyl) trifluoroacétamide.

2. Le procédé selon la revendication 1, **caractérisé en ce que** l'agent de silylation est le bis (triméthylsilyl) acétamide.

3. Le procédé selon les revendications 1 ou 2, **caractérisé en ce que** la base est choisie dans le groupe comprenant la pyridine, l'éthyl diisopropylamine, la triéthylamine et l'éthyl nicotinate.

4. Le procédé selon la revendication 3, **caractérisé en ce que** la base est la pyridine.
